# EUROPEAN PATENT SPECIFICATION

(11) **EP 2 185 239 B1**
(45) Date of publication and mention of the grant of the patent: **27.07.2016**
(21) Application number: 08797683.3
(22) Date of filing: 12.08.2008
(51) Int. Cl.: A61N 1/372

(54) **ROTATING FIELD INDUCTIVE DATA TELEMETRY AND POWER TRANSFER IN AN IMPLANTABLE MEDICAL DEVICE SYSTEM**
DREHFELDINDUKTIVE DATENTELEMETRIE UND LEISTUNGSÜBERTRAGUNG IN EINEM IMPLANTIERBAREN MEDIZINISCHEN VORRICHTUNGSSYSTEM
TÉLÉMESURE DE DONNÉES INDUITES PAR UN CHAMP TOURNANT ET TRANSFERT DE PUISSANCE DANS UN SYSTÈME DE DISPOSITIF MÉDICAL IMPLANTABLE

(30) Priority: 11.09.2007 US 853624
(43) Date of publication of application: 19.05.2010
(73) Proprietor: Boston Scientific Neuromodulation Corporation, Valencia, CA 91355 (US)
(72) Inventor: STOUFFER, Thomas, Warren, Chatsworth CA 91311 (US); FREIDIN, Lev, Simi Valley CA 93603 (US); AGHASSIAN, Daniel, Los Angeles CA 90027 (US)
(74) Representative: Vossius & Partner Patentanwälte Rechtsanwälte mbB
(86) International application number: PCT/US2008/072879
(87) International publication number: WO 2009/035806

(56) References cited:
- US-A- 6 141 588
- US-A1- 2002 147 388
- US-A1- 2005 088 357
- US-A1- 2006 085 041
- US-A1- 2006 195 162
- US-A1- 2008 172 109

## Description

### CROSS REFERNCE TO RELATED APPLICATIONS

This is an International (PCT) application claiming priority to U.S. Patent Application Serial No. 11/853,624, filed September 11, 2007.

### FIELD OF THE INVENTION

The present invention relates to a data telemetry and/or power transfer technique having applicability to implantable medical device systems.

### BACKGROUND

Implantable stimulation devices are devices that generate and deliver electrical stimuli to body nerves and tissues for the therapy of various biological disorders, such as pacemakers to treat cardiac arrhythmia, defibrillators to treat cardiac fibrillation, cochlear stimulators to treat deafness, retinal stimulators to treat blindness, muscle stimulators to produce coordinated limb movement, spinal cord stimulators to treat chronic pain, cortical and deep brain stimulators to treat motor and psychological disorders, and other neural stimulators to treat urinary incontinence, sleep apnea, shoulder sublaxation, etc. The present invention may find applicability in all such applications, although the description that follows will generally focus on the use of the invention within a Spinal Cord Stimulation (SCS) system, such as that disclosed in U.S. Patent 6,516,227.

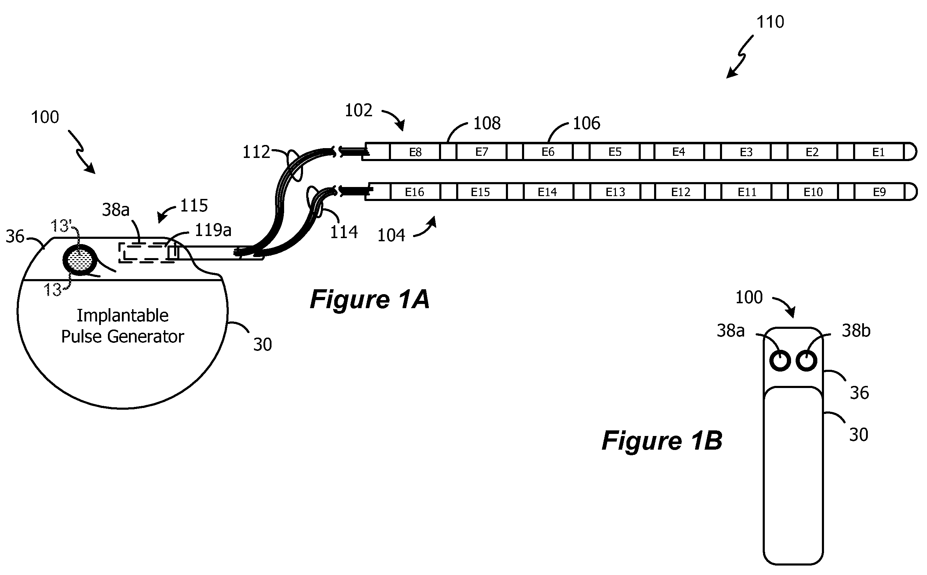

Spinal cord stimulation is a well-accepted clinical method for reducing pain in certain populations of patients. As shown in Figures 1A and 1B, a SCS system typically includes an Implantable Pulse Generator (IPG) 100, which includes a biocompatible case 30 formed of titanium for example. The case 30 typically holds the circuitry and power source or battery necessary for the IPG to function, although IPGs can also be powered via external RF energy and without a battery. The IPG 100 is coupled to electrodes 106 via one or more electrode leads (two such leads 102 and 104 are shown), such that the electrodes 106 form an electrode array 110. The electrodes 106 are carried on a flexible body 108, which also houses the individual signal wires 112 and 114 coupled to each electrode. In the illustrated embodiment, there are eight electrodes on lead 102, labeled E₁-E₈, and eight electrodes on lead 104, labeled E₉-E₁₆, although the number of leads and electrodes is application specific and therefore can vary.

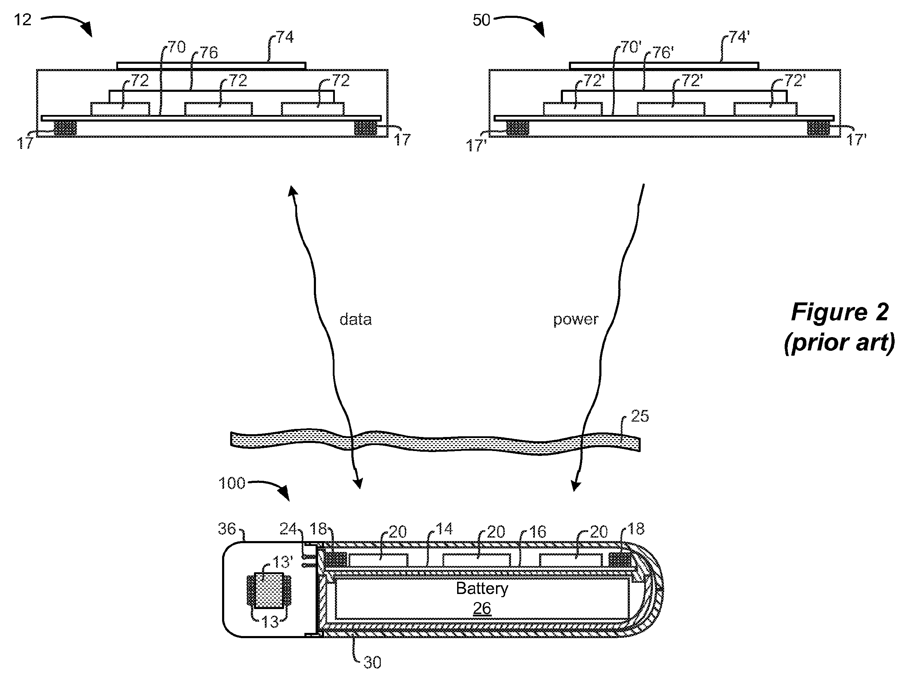

As shown in Figure 2, the IPG 100 typically includes an electronic substrate assembly 14 including a printed circuit board (PCB) 16, along with various electronic components 20, such as microprocessors, integrated circuits, and capacitors mounted to the PCB 16. Two coils are generally present in the IPG 100: a telemetry coil 13 used to transmit/receive data to/from an external controller 12; and a charging coil 18 for charging or recharging the IPG's power source or battery 26 using an external charger 50. The telemetry coil 13 can be mounted within the header connector 36 as shown.

As just noted, an external controller 12, such as a hand-held programmer or a clinician's programmer, is used to wirelessly send data to and receive data from the IPG 100. For example, the external controller 12 can send programming data to the IPG 100 to dictate the therapy the IPG 100 will provide to the patient. Also, the external controller 12 can act as a receiver of data from the IPG 100, such as various data reporting on the IPG's status. The external controller 12, like the IPG 100, also contains a PCB 70 on which electronic components 72 are placed to control operation of the external controller 12. A user interface 74 similar to that used for a computer, cell phone, or other hand held electronic device, and including touchable buttons and a display for example, allows a patient or clinician to operate the external controller 12. The communication of data to and from the external controller 12 is enabled by a coil 17, which is discussed further below.

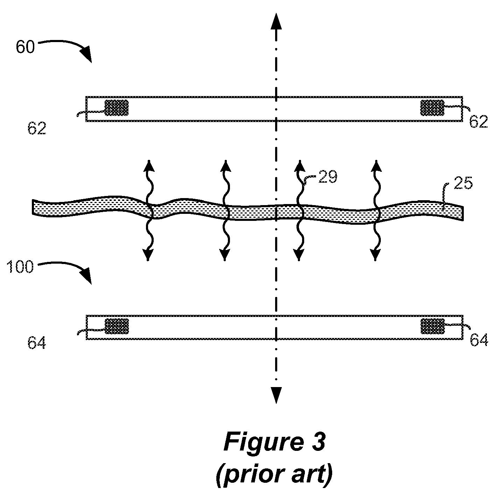

The external charger 50, also typically a hand-held device, is used to wirelessly convey power to the IPG 100, which power can be used to recharge the IPG's battery 26. The transfer of power from the external charger 50 is enabled by a coil 17', which is discussed further below. For the purpose of the basic explanation here, the external charger 50 is depicted as having a similar construction to the external controller 12, but in reality they will differ in accordance with their functionality as one skilled in the art will appreciate. However, given the basic similarities between the external controller 12 and the external charger 50 as concerns this disclosure, they are depicted as a single external device 60 in Figure 3.

Wireless data transfer and/or power transfer between the external device 60 and the IPG 100 takes place via inductive coupling, and specifically magnetic inductive coupling. To implement such functionality, and as alluded to above, both the IPG 100 and the external device 60 have coils which act together as a pair. When the external device 60 is an external controller 12, the relevant pair of coils comprises coil 17 from the controller and coil 13 from the IPG. When the external device 60 is an external charger 50, the relevant pair of coils comprises coil 17' from the external charger and coil 18 from the IPG. In the generic external device 60 depicted in Figure 3, only one coil pair is depicted for simplicity, namely coil 62 from the external device 60 (which can comprise either coil 17 or 17'), and coil 64 from the IPG 100 (which can comprise either coil 13 or 18). Either coil 62 or 64 can act as the transmitter or the receiver, thus allowing for two-way communication between the external device 60 and the IPG 100.

When data is to be sent from the external device 60 to the IPG 100 for example, coil 62 is energized with an alternating current (AC). Such energizing of the coil 62 to transfer data can occur using a Frequency Shift Keying (FSK) protocol for example, such as disclosed in U.S. Patent Application Serial No. 11/780,369, filed July 19, 2007, which is incorporated herein by reference in its entirety. Energizing the coil 62 induces an electromagnetic field 29, which in turn induces a current in the IPG's coil 64, which current can then be demodulated to recover the original data.

When power is to be transmitted from the external device 60 to the IPG 100, coil 62 is again energized with an alternating current. Such energizing is generally of a constant frequency, and of a larger magnitude than that used during the transfer of data, but otherwise the physics involved are similar.

Regardless of whether the external device 60 is transferring data or power, the energy used to energize the coil 62 can come from a battery in the external device 60 (not shown in Fig. 3), which like the IPG's battery 26 is preferably rechargeable. However, power may also come from plugging the external device 60 into a wall outlet plug (not shown), etc.

As is well known, inductive transmission of data or power can occur transcutaneously, i.e., through the patient's tissue 25, making it particular useful in a medical implantable device system. During the transmission of data, the coils 62 and 64 preferably lie in planes that are parallel, along collinear axes, and with the coils in as close as possible to each other, such as is shown generally in Figure 3. Such an orientation between the coils 62 and 64 will generally improve the coupling between them, but deviation from ideal orientations can still result in suitably reliable data or power transfer.

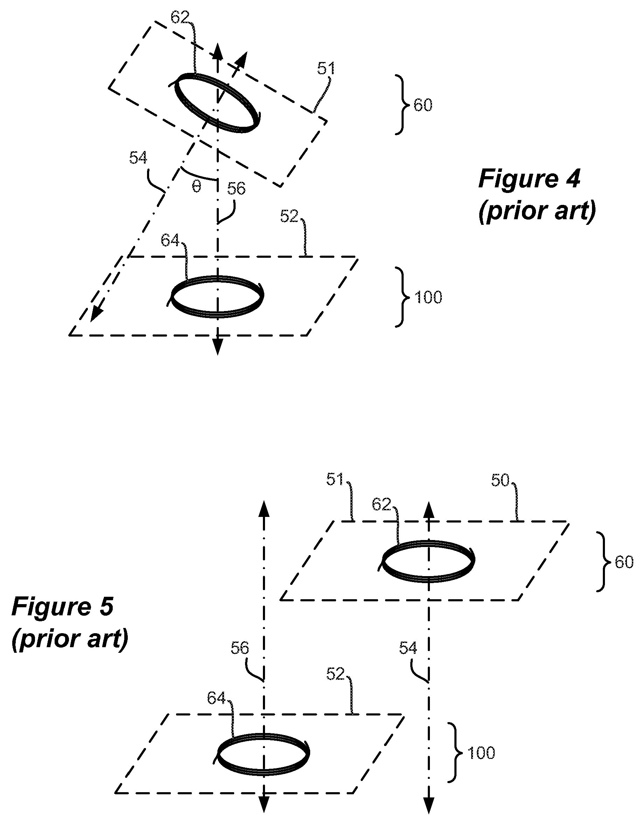

However, realization of this ideal orientation condition necessarily relies on successful implementation by the user of the external device 60. For example, and as shown in Figure 4, if the angle θ between the axis 54 of coil 62 and the axis 56 of coil 60 is non-ideal (i.e., non-zero), data or power transfer will be non-ideal. When the axes 54, 56, are perpendicular, theoretically no energy will be transferred, and realistically only a negligible amount of energy will be transferred. Another non-ideal orientation between coil 62 and coil 60 is shown in Figure 5. In this instance, the axes 54 and 56 of the coils are parallel, as are their planes 51 and 52, but they are not colinear, with the result that the coils are not overlapping. This too adversely impacts the coupling from coil 62 to coil 64.

The non-ideal orientations depicted in Figures 4 and 5 illustrate that a user of an external device 60 must be attentive to proper placement of that device relative to the IPG 100. Requiring correct placement by the user is of course a drawback of such traditional IPG system hardware, because it is unrealistic to assume that any given user will be so attentive, and as a result data or power transfer may be adversely affected.

Further exacerbating the potential problem of improper external device-to-IPG orientation is the recognition that improper orientations are not necessarily always the result of user inadvertence. It has so far been assumed that it is relatively easy for the user to understand or infer the positioning of the coils 62 and 64. For example, when both the external device 60 and the IPG 100 are basically flat, placing the coils 62, 64 close to the ideal orientation depicted in Figure 3 is not difficult. But what if the external device 60 or IPG 100 is not flat? What if the coils are mounted inside the housings in a manner in which the coil position cannot be inferred? What if the IPG 100 is implanted deep within a patient, such that the orientation of its coil 62 cannot be inferred through the patient's tissue? What if the IPG 100 moves or rotates within the patient after it is implanted? Any of these effects can make it difficult or impossible for even an attentive user to properly align the coil 62 in the external device 60 and the coil 64 in the IPG 100.

From the foregoing, it should be clear that the art of magnetically-coupled implantable medical device systems would benefit from improved techniques for ensuring good coupling between the external device and the IPG, even during conditions of non-ideal alignment. This disclosure provides embodiments of such a solution. Document US-A-6 141 588 discloses the most relevant prior art. The invention is defined in claim 1.

### BRIEF DESCRIPTION OF THE DRAWINGS

Figures 1A and 1B show an implantable pulse generator (IPG), and the manner in which an electrode array is coupled to the IPG in accordance with the prior art.
Figure 2 shows wireless communication of data between an external controller and an IPG, and wireless communication of power from an external charger to the IPG.
Figure 3 generalizes the external controller and the external charge to a single external device.
Figures 4 and 5 show types of non-ideal orientations between the external device and the IPG which result in poor coupling, and hence poor data and power transfer.
Figure 6 shows an embodiment of the disclosed dual transmitter coil approach, in which orthogonal dual coils are used in the transmitter of the external device-IPG system.
Figure 7 and 8 show the transmitter circuitry used in the transmitter, and shows that the two coils are driven with the broadcast data with an approximately 90 degree phase difference.
Figure 9 shows in the internal structure of an external device including the dual transmitter coils.
Figure 10 shows receiver circuitry useable in a device using dual transmitter coils.

### DETAILED DESCRIPTION

The description that follows relates to use of the invention within a spinal cord stimulation (SCS) system. However, the invention is not so limited. Rather, the invention may be used with any type of implantable medical device system that could benefit from improved coupling between an external device and the implanted device. For example, the present invention may be used as part of a system employing an implantable sensor, an implantable pump, a pacemaker, a defibrillator, a cochlear stimulator, a retinal stimulator, a stimulator configured to produce coordinated limb movement, a cortical and deep brain stimulator, or in any other neural stimulator configured to treat any of a variety of conditions.

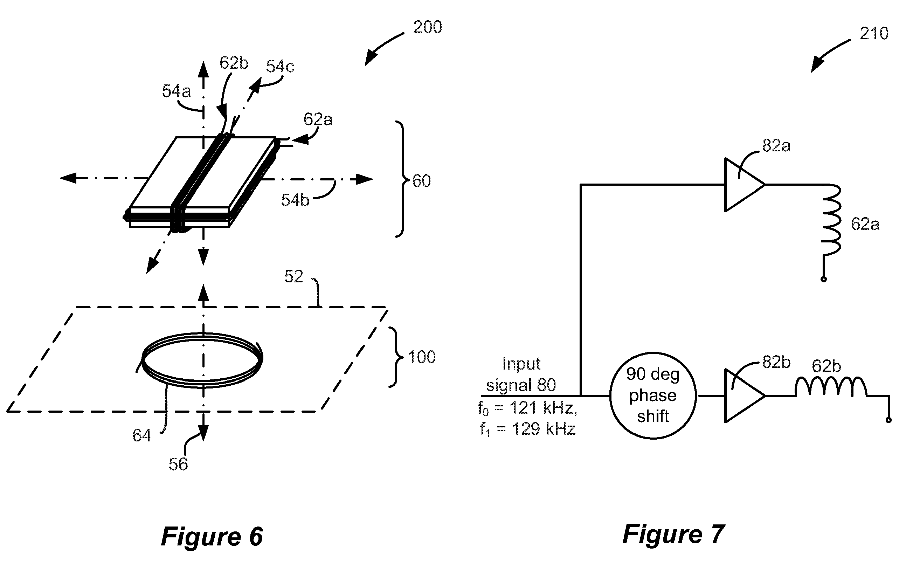

As shown in the simplified illustration of Figure 6, the disclosed improved implantable medical device system 200 uses dual coils 62a and 62b in the transmitting device. In a preferred implementation, the dual coils 62a and 62b are included in the external device 60 as the transmitter, although the dual coils could also be included in the IPG to improve its ability to back telemeter status data. When the dual coils 62a and 62b are included in the external device 60, the external device is most preferably the external controller 12, but could also comprise the external charger 50 (see Fig. 2). For simplicity sake, and without intention to limit the technique, the foregoing discussion describes an embodiment employing these preferences in which the dual transmitting coils are employed in an external controller for improved data transfer.

As shown in Figure 6, the dual coils 62a and 62b are respectively wrapped around axes 54a and 54b which are preferably orthogonal, i.e., the angle between axes 54a and 54b is preferably 90 degrees. However, this is not strictly necessary, and the disclosed technique improves over the prior art if any non-zero angle is used between the axes 54a and 54b. That being said, maximal benefit is achieved when this angle approaches 90 degrees, i.e., approximately 90 as close as mechanical tolerances will allow.

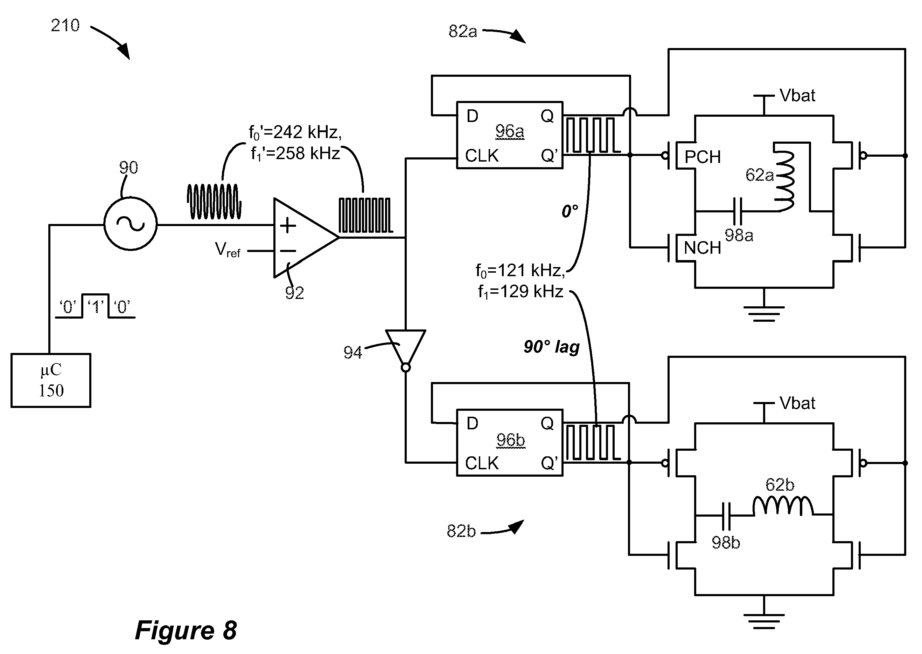

Figures 7 and 8 depict the transmitter circuitry 210 used to drive the two coils 62a and 62b. Figure 7 describes such circuitry in a basic block diagram form, while Figure 8 shows further details as presently preferred in an actual implementation. In either case, it should be understood that other details of the transmitter circuitry are not set forth for clarity, but are well known.

As shown in Figure 7, the two coils 62a and 62b are driven with the same signal but out of phase, and most preferably with a 90 degree phase shift between them. For example, consider an application in which the dual coils 62a and 62b are used in an external controller to serially telemeter data bits to the IPG 100. Those signals are centered around f_{c} = 125 kHz, with a logic '1 bit being represented by an approximately 129kHz input signal 80 (f₁), and a logic '0' bit being represented by an approximately 121 kHz input signal 80 (f₀). (Such an example illustrates the use of FSK modulation, which is described in further detail in the above-incorporated '369 application). This modulated input signal 80 is split, and is phase shifted by approximately 90 degrees (i.e., by 1 / (4 * f_{c}), or 2 microseconds) in the leg that goes to the driver 82b for the coil 62b. This phase shift in the lower leg to coil 62b can comprise either a 90 degree lag or a 90 degree lead when compared to the signal in the top leg to coil 62a; however, for ease of discussion, a lagging signal is illustrated herein. It should be realized that the phase shift between the two legs is approximately 90 degrees, with the actual angle between them depending on the particular frequency (f₀ or f₁) being processed at any given time.

Figure 8 discloses a more detailed schematic for transmitter circuitry 210 in a preferred embodiment. Generation of the driving signals for the two coils 62a and 62b starts with the external device's microcontroller 150, preferably Part No. MSP430 manufactured by Texas Instruments, Inc. The microcontroller 150 outputs a string of digital data bits that are ultimately to be wirelessly broadcast using the transmitter circuitry 210. The digital data is sent to modulation circuitry (oscillator) 90, preferably Part No. AD9834 manufactured by Analog Devices, Inc. The oscillator 90 converts the digital bits to AC waveforms whose frequency depends on the logic state of the particular bit being processed (again, as is consistent with use of an FSK protocol). In this embodiment, the center frequency f_{c}' as output by the oscillator 90 is 250 kHz, or twice the desired center frequency f_{c}=125 kHz to be ultimately broadcast by the transmitter circuitry 210. When modulated with the logic states, the result is an AC output of either f₀'=242 kHz or f₁'=258 kHz. This AC output is then turned into a square wave of the same frequency by a comparator 92 as one skilled in the art will appreciate.

Thereafter, the modulated square wave data signal is split into two legs that ultimately drive the two coils 62a and 62b. Each leg receives the square wave output at a clocking input (CLK) of DQ flip flops 96a and 96b, although the data received at the lower leg is inverted by an inverter 94. The inverter essentially works a 180 degree shift in the square wave data signal. The complimentary output Q' of each flip flop 96a and 96b is coupled to the corresponding input D. Given this arrangement, and appreciating that the flip flops 96a and 96b can only change data states upon a rising edge of its clock input, the effect is that the outputs (Q/Q') of the flip flops 96a and 96 comprise a square wave signal at half the frequency (i.e., frequencies of f₀= 121 kHz and f₁ = 129 kHz), but in which the signal driving the lower leg lags by 90 degrees. This approximately 90 degree shift in the lower frequency (f_{c} = 125 kHz) signal stems from the approximately 180 degree shift imparted by the inverter 94 at the higher frequency (f_{c}'=250 kHz) signal.

The lower frequency square wave signals are in turn used to resonant the coils 62a and 62b, again, with the signals arriving at coil 62b with a 90 degree lag. Resonance is achieved for each coil 62a and 62b through a serial connection to a tuning capacitor 98a, 98b, making a resonant LC circuit. As one skilled in the art will appreciate, the N-channel (NCH) and P-channel (PCH) transistors are gated by either the output (Q) or the complementary output (Q') of the flip flops 96a and 96b to apply the voltage, Vbat, needed to energize the coils 62a and 62b. Such voltage Vbat comes from the battery (or other power source) with the external device 60. One skilled in the art will appreciate that the disclosed arrangement reverses the polarity of this battery voltage Vbat across the series-connected LC circuit (+Vbat followed by -Vbat followed by +Vbat, etc.), which in turn causes the coils to resonate and therefore broadcast at the frequencies of interest (f₀ = 125 kHz; f₁ = 129 kHz). It should be understood that transmitter circuitry 210 as depicted in Figure 8 could be made in different ways, and therefore what is disclosed is merely one non-limiting example.

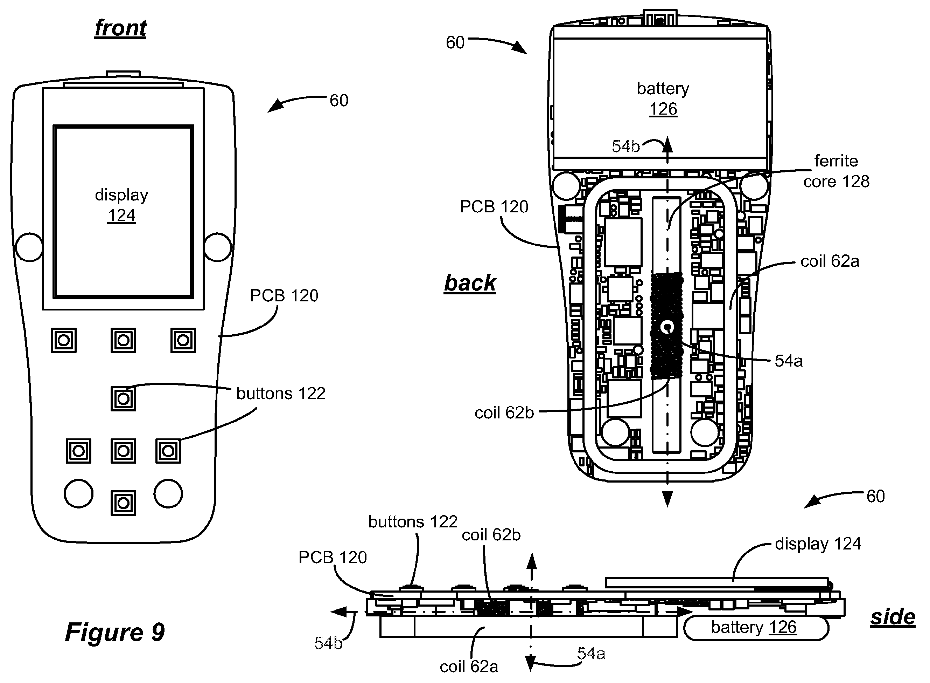

Figure 9 shows the structure of an external device 60 and the physical orientation of the coils 62a and 62b as well as some of the other components. As envisioned, the external device 60 as depicted comprises an external controller, but could also comprises an external charger (see Fig. 2). So that the internal components can be more easily seen, the external device (controller) 60 is depicted without its outer housing, and from front, back, and side perspectives.

As shown, the external device (controller) 60 comprises a printed circuit board (PCB) 120, whose front side carries the user interface, including a display 124 and buttons 122. The operative circuitry, including the coils 62a and 62b and the battery 126, are located on the back side of the PCB 120, along with other integrated and discrete components necessary to implement the functionality of the external controller. As seen in the back and side views, the two coils 62a and 62b are respectively wrapped around axes 54a and 54b which are orthogonal. Coil 62a is wrapped in a racetrack configuration around the back of the PCB 120, while coil 62b is wrapped around a ferrite core 128 and affixed to the PCB 120 by epoxy.

With the transmitter circuitry 210 and the physical construction of the external device (controller) 60 set forth, the theory of operation of the device is briefly explained. By causing the input signals to the two coils to be 90 degrees out of synchronization, the magnetic field produced by the two coils will effectively rotate around a third axis 54c (Fig. 6) orthogonal to both of the coils' axes 54a and 54b. The effect can be analogized to a bar magnet spinning around axis 54c with an angular velocity of either f₀ (121 kHz) or f₁ (129 kHz) depending on the data state being transmitted at any given time. Because the produced magnetic field spins, the number and severity of nulls in the magnetic field are reduced at the receiving coil 64 in the IPG 100. In fact, the only significant null condition exists when the axes of the spinning field 54c and the axis of the receiving coil 56 are aligned (not shown in Fig. 6). As a result, the system is not dependent on user attentiveness to provide suitable coupling between the coils 62a and 62b in the external device 60 and the coil 64 in the IPG 100, with the result that the reliability of data or power transfer is improved.

Fortunately, use of the disclosed dual-coil technique does not require any changes in the receiver circuitry used in conjunction with the receiving coil 64 within the IPG 100. This results from the understanding that current can be induced in the receiving coil 64 either by changing the magnitude of the produced magnetic field (as occurs in traditional signal transmitter coil systems), or by changing the direction of the magnetic field (as occurs with the disclosed dual transmitter coil technique). In either case, one skilled in the art should appreciate that Faraday's law illustrates that the current induced in the receiving coil will be equivalent whether a single transmitter coil is used, or two orthogonal transmitter coils are used but driven 90 degrees out of phase. This assumes however that each of the coils 62a and 62b in the dual-coil system are capable of generating a magnetic field of the same strength as that produce by the singular coil in a single coil system. Designing the coils 62a and 62b (number of turns, etc.) and the transmitter circuitry 210 to achieve equal magnetic strength from the two contributing magnetic fields is therefore desirable, but not absolutely necessary. The benefits of the use of dual transmitter coils are still realized even if the coils do not contribute equally to the produced magnetic field.

From the foregoing, and because of the desire to maintain a consistent magnitude of induced current in the receiving coil, the disclosed dual coil approach may take more power (e.g., twice the power) than approaches using single coils. This additional power requirement is generally not problematic, as the battery power within the external device is not critical and can be easily recharged during periods in which the external device 60 is not used. In any event, it is clearly beneficial that implementation of the dual-coil technique does not require any re-tooling of the IPG or its receiver circuitry.

While the receiver circuitry in the IPG 100 does not require modification, the receiver circuitry in the external device 60 may be changed to account for the two coils 62a and 62b, assuming that such coils are used as the antennas for so-called "back telemetry" (e.g., status data) received from the IPG 100. (Obviously, the external device 60 would contain no receiver circuitry in an IPG system lacking back telemetry capability).

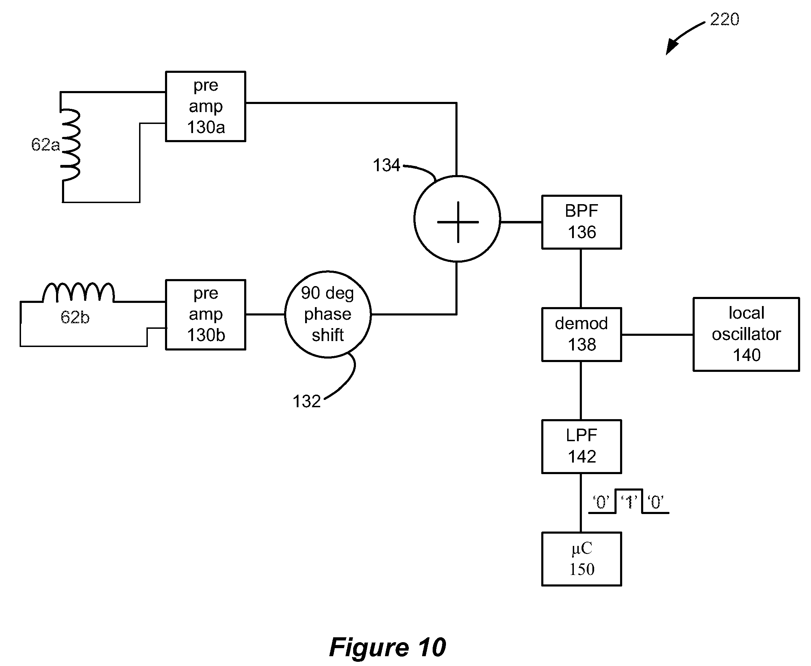

Exemplary receiver circuitry 220 useable with the dual coils 62a and 62b in the external device 60 and for receiving a wireless modulated data signal from the IPG 100 is shown in Figure 10. As with the transmitter circuitry 210 (Figs. 7 and 8) the receiver circuitry 220 comprises two legs coupled to each of the two coils. Pre-amplifiers (pre-amps) 130a and 130b initially amplify the received modulated signals from the two coils 62a and 62b respectively. Thereafter, the amplified signal from pre-amp 130b is shifted 132 by 90 degrees, which shift can be imparted by any number of circuitry approaches as one skilled in the art will appreciate. As with the transmitter circuit 210, this phase shift 132 can comprise either a lagging or leading of the comparable signal as received from coil 62a; a delay is preferred because it is easier to implement.

Thereafter, the amplified signals, with the phase shift applied between them, are added together at a summer circuit 134, which again can comprise any well known analog summer circuitry known in the art. The resulting signal is then subject to a band pass filter (BPF) 136, which removes frequencies component from the signal outside of the frequency band of interest (e.g., outside of the range from 121 to 129 kHz). This signal is then demodulated back into digital bits at a demodulator block 138 operating under the control of a local oscillator 140. Noise is removed from these digital bits at a low pass filter block 142, which then allows the received data to be input to the external controller's microcontroller 150 for interpretation and processing. One skilled in the art will appreciate that summer 134, the BPF 136, demodulation block 138, local oscillator 140, and LPF 142, or any combination of these blocks, can collectively comprise demodulation circuitry.

Receiver circuitry 220 of Figure 10 is not the only manner in which data can be received at the two coils 62a and 62b. For example, during data reception periods, each antenna (coil) 62a and 62b could be sequentially monitored during a preamble portion of the communication protocol to assess the signal quality at each antenna coil. Thereafter, the coil 62a or 62b with the best signal quality could be used for reception, with the other coil disconnected during the remainder of the data reception period.

Other embodiments of the invention can be varied from the preferred embodiments disclosed. For example, and as noted earlier, neither the physical angle between the axes 54a and 54b of the transmitter coils 62a and 62b, nor the phase angle between the signal driving them, need be exactly 90 degrees.

While disclosed in the context of a medical implantable device system for which the invention was originally contemplated, it should be recognized that the improved dual-coil approach herein is not so limited, and can be used in other contexts employing communications via magnetic inductive coupling, such as in Radio-Frequency Identification (RFID) systems, etc. The disclosed circuitry can further be used in any context in which magnetic inductive coupling could be used as a means of communication, even if not so used before.

Although particular embodiments of the present invention have been shown and described, it should be understood that the above discussion is not intended to limit the present invention to these embodiments. It will be obvious to those skilled in the art that various changes and modifications may be made without departing from the spirit and scope of the present invention. Thus, the present invention is intended to cover alternatives, modifications, and equivalents that may fall within the scope of the present invention as defined by the claims.

## Claims

1. An external device (60) useable to transfer power or data to an implantable medical device, comprising:
a printed circuit board (120) having a front side and a back side, wherein the front side of the printed circuit board (120) carries a user interface;
a first coil (62a) formed in a first plane parallel to the printed circuit board (120) and comprising a plurality of turns wrapped around a first axis perpendicular to the first plane, the first coil (62a) being wrapped in a racetrack configuration around the back of the printed circuit board (120);
a second coil (62b) comprising a plurality of turns wrapped around a second axis, wherein the first axis is orthogonal to the second axis, and wherein the second coil (62b) is wrapped around a ferrite core (128) and affixed to the printed circuit board (120);
transmitter circuitry configured to produce a signal to drive the first and second coils (62a, 62b); and
a battery (126);
wherein the first and second coils (62a, 62b), the transmitter circuitry and the battery (126) are located on the back side of the printed circuit board (120); and
wherein the signal is phase shifted at one of the coils when compared to the other coil to produce a rotating magnetic field for transferring the power or data to the implantable medical device.

2. The device of claim 1, wherein the magnetic field rotates around a third axis, wherein the third axis is preferably orthogonal to the first and second axes.

3. The device of claim 1, wherein the signal comprises a modulated data signal, wherein the modulated data signal is preferably modulated using a Frequency Shift Keying protocol.

4. The device of claim 1, wherein the first and second coils are coupled to receiver circuitry to receive a wireless broadcast from the implantable medical device.

5. The device of claim 1, further comprising a housing, wherein the housing contains the printed circuit board (120), the first and second coils, and the transmitter circuitry, wherein the housing is preferably hand-held.

6. The device of claim 1, wherein the signal is phase shifted at one of the first and second coils when compared to the other of the first and second coils by 90 degrees.

7. The device of claim 1, wherein the user interface comprises a display and buttons.

8. The device of claim 4, wherein the receiver circuitry comprises:
a summer configured to receive first and second signals from the first and second coils respectively, wherein the summer adds the received first and second signals, and
demodulation circuitry coupled to the output of the summer;
wherein one of the first and second signals is a phase shifted signal with respect to the other of the first and second signals prior to being received at the summer.

## Patentansprüche

1. Externe Vorrichtung (60), die dazu verwendbar ist, Energie oder Daten zu einem implantierbaren Medizinprodukt zu übertragen und die aufweist:
eine Leiterplatte (120) mit einer Vorderseite und einer Rückseite, wobei die Vorderseite der Leiterplatte (120) eine Benutzerschnittstelle trägt;
eine erste Spule (62a), die in einer ersten Ebene parallel zur Leiterplatte (120) gebildet ist und mehrere Windungen aufweist, die um eine erste Achse senkrecht zur ersten Ebene gewickelt sind, wobei die erste Spule (62a) in einer Rennbahnkonfiguration um die Rückseite der Leiterplatte (120) gewickelt ist;
eine zweite Spule (62b), die mehrere Windungen aufweist, die um eine zweite Achse gewickelt sind, wobei die erste Achse orthogonal zur zweiten Achse ist und wobei die zweite Spule (62b) um einen Ferritkern (128) gewickelt und an der Leiterplatte (120) befestigt ist;
einen Senderschaltungsaufbau, der so konfiguriert ist, dass er ein Signal erzeugt, um die erste und zweite Spule (62a, 62b) anzusteuern; und
eine Batterie (126);
wobei die erste und zweite Spule (62a, 62b), der Senderschaltungsaufbau und die Batterie (126) auf der Rückseite der Leiterplatte (120) liegen; und
wobei das Signal an einer der Spulen verglichen mit der anderen Spule phasenverschoben ist, um ein magnetisches Drehfeld zum Übertragen der Energie oder der Daten zum implantierbaren Medizinprodukt zu erzeugen.

2. Vorrichtung nach Anspruch 1, wobei das magnetische Feld um eine dritte Achse dreht, wobei die dritte Achse vorzugsweise orthogonal zur ersten und zweiten Achse ist.

3. Vorrichtung nach Anspruch 1, wobei das Signal ein moduliertes Datensignal auf weist, wobei das modulierte Datensignal vorzugsweise mit Hilfe eines Frequenzumtastungsprotokolls moduliert ist.

4. Vorrichtung nach Anspruch 1, wobei die erste und zweite Spule mit einem Empfängerschaltungsaufbau gekoppelt sind, um eine drahtlose Übertragung vom implantierbaren Medizinprodukt zu empfangen.

5. Vorrichtung nach Anspruch 1, die ferner ein Gehäuse aufweist, wobei das Gehäuse die Leiterplatte (120), die erste und zweite Spule und den Senderschaltungsaufbau enthält, wobei das Gehäuse vorzugsweise tragbar ist.

6. Vorrichtung nach Anspruch 1, wobei das Signal an einer der ersten und zweiten Spule verglichen mit der anderen der ersten und zweiten Spule 90 Grad phasenverschoben ist.

7. Vorrichtung nach Anspruch 1, wobei die Benutzerschnittstelle ein Display und Tasten aufweist.

8. Vorrichtung nach Anspruch 4, wobei der Empfängerschaltungsaufbau aufweist:
einen Addierer, der so konfiguriert ist, dass er ein erstes und ein zweites Signal von der ersten bzw. zweiten Spule empfängt, wobei der Addierer das empfangene erste und zweite Signal addiert, und
einen Demodulationsschaltungsaufbau, der mit dem Ausgang des Addierers gekoppelt ist;
wobei eines des ersten und zweiten Signals im Hinblick auf das andere des ersten und zweiten Signals vor Empfang am Addierer ein phasenverschobenes Signal ist.

## Revendications

1. Dispositif externe (60) utilisable en vue de transférer une puissance ou des données à un dispositif médical implantable, comprenant :
une carte de circuit imprimé (120) présentant une face avant et une face arrière, dans lequel la face avant de la carte de circuit imprimé (120) porte une interface utilisateur ;
un premier enroulement (62a) formé dans un premier plan parallèle à la carte de circuit imprimé (120) et comprenant une pluralité de spires enroulées autour d'un premier axe perpendiculaire au premier plan, le premier enroulement (62a) étant enroulé dans une configuration de piste autour de l'arrière de la carte de circuit imprimé (120) ;
un second enroulement (62b) comprenant une pluralité de spires enroulées autour d'un deuxième axe, dans lequel le premier axe est orthogonal au deuxième axe, et dans lequel le second enroulement (62b) est enroulé autour d'un noyau de ferrite (128) et fixé à la carte de circuit imprimé (120) ;
un montage de circuits d'émission configuré de manière à produire un signal en vue de commander les premier et second enroulements (62a, 62b) ; et
une batterie (126) ;
dans lequel les premier et second enroulements (62a, 62b), le montage de circuits d'émission et la batterie (126) sont situés sur la face arrière de la carte de circuit imprimé (120) ; et
dans lequel le signal est déphasé au niveau d'un des enroulements par rapport à l'autre enroulement en vue de produire un champ magnétique tournant pour transférer la puissance ou les données au dispositif médical implantable.

2. Dispositif selon la revendication 1, dans lequel le champ magnétique tourne autour d'un troisième axe, dans lequel le troisième axe est de préférence orthogonal aux premier et deuxième axes.

3. Dispositif selon la revendication 1, dans lequel le signal comprend un signal de données modulé, dans lequel le signal de données modulé est de préférence modulé en utilisant un protocole de modulation par déplacement de fréquence.

4. Dispositif selon la revendication 1, dans lequel les premier et second enroulements sont couplés à un montage de circuits de réception en vue de recevoir une diffusion sans fil en provenance du dispositif médical implantable.

5. Dispositif selon la revendication 1, comprenant en outre un boîtier, dans lequel le boîtier contient la carte de circuit imprimé (120), les premier et second enroulements et le montage de circuits d'émission, dans lequel le boîtier est de préférence portatif.

6. Dispositif selon la revendication 1, dans lequel le signal est déphasé au niveau de l'un des premier et second enroulements par rapport à l'autre des premier et second enroulements de 90 degrés.

7. Dispositif selon la revendication 1, dans lequel l'interface utilisateur comprend un affichage et des boutons.

8. Dispositif selon la revendication 4, dans lequel le montage de circuits de récepteur comprend :
un sommateur configuré de manière à recevoir des premier et second signaux en provenance des premier et second enroulements, respectivement, dans lequel le sommateur additionne les premier et second signaux reçus ; et
un montage de circuits de démodulation couplé à la sortie du sommateur ;
dans lequel l'un des premier et second signaux est un signal déphasé par rapport à l'autre des premier et second signaux avant d'être reçu au niveau du sommateur.
